# EUROPEAN PATENT SPECIFICATION

(11) **EP 0 304 494 B1**
(45) Date of publication and mention of the grant of the patent: **17.11.1994**
(21) Application number: 88902542.5
(22) Date of filing: 11.03.1988
(51) Int. Cl.: G01N 27/30, G01N 27/416, G01N 27/414

(54) **IMMOBILIZATION OF BIOFUNCTIONAL MATERIAL, ELEMENT PREPARED THEREFROM AND MEASUREMENT USING THE SAME**
IMMOBILISIERUNG VON BIOFUNKTIONELLEM MATERIAL, DARAUS ERZEUGTES ELEMENT UND MASSNAHME ZU DESSEN VERWENDUNG
IMMOBILISATION DE MATIERE BIOFONCTIONNELLE, ELEMENT PREPARE A PARTIR DE CETTE MATIERE ET MESURE L'UTILISANT

(30) Priority: 12.03.1987 JP 55387/87; 03.12.1987 JP 304524/87
(43) Date of publication of application: 01.03.1989
(73) Proprietor: JAPAN, as represented by PRESIDENT OF NATIONAL REHABILITATION CENTER FOR THE DISABLED, Tokorozawa-shi, Saitama 359 (JP); SUMITOMO CEMENT CO. LTD., Chiyoda-ku Tokyo 101 (JP)
(72) Inventor: IKARIYAMA, Yoshihito, Tokorozawa-shi Saitama 359 (JP); YAMAUCHI, Shigeru, Chiyoda-ku Tokyo 102 (JP); IKUASHI, Tomoaki, Narashino-shi Chiba 275 (JP)
(74) Representative: Bühling, Gerhard, Dipl.-Chem.
(86) International application number: PCT/JP88/00256
(87) International publication number: WO 88/07193

(56) References cited:
- JP-A-56 163 447
- Reference Electrodes, ed. D.J.G. Ives and G.J. Janz, Academic Press (N.-Y. and London), 1961, pages 103-111

## Description

### Method of Immobilizing Biofunctional Material, and Element prepared thereby and, Measurement by Using the same Element

### FIELD OF ART

The present invention relates to a method of immobilizing a biologically active (biofunctional) substance, to the element prepared thereby, and to a method of measurement by use of the element. Particularly it relates to a technique for miniaturizing a biologically active electrode in use for quick and sensitive measurement of a specified substance, and to a detecting element that can be prepared by this technique.

Further the present invention relates to a analytical apparatus and an analytical method using the same element. Particularly, it relates to a noble microelectrode element prepared thereby, and the analytical apparatus and an analytical method using the electrode, with highly rapid response and high sensitivity and high detecting output.

### BACKGROUND OF THE ART

There have been known that biosensor prepared by the incorporation of enzymes, antibody or microorganisms on the surface of platinum or carbon can enable to measure quickly and continuously various chemical substances and biologically active substances. In such preparation, the biologically active substances are applied on the surface of the electrode by preparing independently the membrane containing the biologically active substance, and adhering the membrane on the surface of the electrode. The other preparation is carried out by forming covalent bond between enzyme and the surface. However, while the feature of biosensor is evaluated by reproducibility, life-period, high sensitivity and response ability, one prepared by the former preparation is not good at reproducibility, and one prepared by the latter preparation is difficult to increase the density of the enzymes on the surface. Further, the prior art preparation needs several steps for the formation of the bioelectrode, and in addition, it is difficult to prepare multifunctional sensor in which several species of biologically active substances on one sensor are incorporated.

The prior art enzyme- immobilized electrode has the structure of enzyme-immobilized membrane adhered on the surface of platinum plate. The preparation thereof may include the adhering of previously prepared enzyme-immobilized membrane on the platinum electrode, and the coating of chemically treated clean surface of the electrode with enzyme. Then, the miniaturization of the electrode is difficult. Recently, the significant technique for miniaturization is a semiconductor integration technique. In such integration technique, enzyme electrode having size in mm order can be considered, but the conventional detecting method by potential measurement will result in satisfaction in sensitivity and response ability. Further, the size of the conventional electrode is difficult to be more miniaturized.

In the prior art, the flow system enzyme analysis comprises flowing a sample to be detected along the passage, detecting by an electrochemical sensor and an enzyme sensor assembled along with the passage to prevent mutual interaction so as to exert the analysis (see Japanese Patent Application Laid-open Publication No.59-133455). Further, there was proposed provision of a functional electrode in use for detecting hazardous substance so as to measure by a biosensor with prevention of trouble.( Japanese Patent Application Laid-open Publication No.61-3047). However, the bioelectrode used for those sensors is the electrode as mentioned below, and has the substantial shortcomings same as in the prior art bioelectrode, and there has not been found the electrode to resolve such problems.

JP-A-56 163 447 describes an enzyme electrode comprising a substrate consisting of a graphite carrier, a platinum layer provided on the surface thereof and an enzyme layer fixed thereto with glutaric aldehyde.

The biosensor in which enzyme, antibody or microorganism is immobilized on the surface of platinum or carbon plate has been known to enable measuring quickly and continuously various chemical or biological substance. In such biosensor, biologically active substance is immobilized by adhering a membrane containing the biologically active substance on the surface of the electrode, or by coating the chemically treated surface of the electrode with enzyme and the like, and forming covalent bond between the surface and the enzyme.

The prior art enzyme embodied electrode has the structure similar to that of Clark type oxygen electrode, or the structure of enzyme embodied film adhered on the surface of the platinum plate. The prior art fabrications of these electrodes may include adhering of the enzyme embodied film on an electrode, and coating of a chemically treated smooth surface of a platinum electrode with enzyme. However, such fabrications cannot enable to make miniaturization. While the recently noted miniaturization technique is a semiconductor integration, the enzyme of several millimeter in diameter can be fabricated by using such technique. Such fabrication using a potential detection does not satisfy demand in phases of sensitivity and response ability. Further, miniaturization to decrease more the size of the electrode would be difficult.

### DISCLOSURE OF INVENTION

The present invention resides in the provision of biosensor system to overcome the shortcomings of the prior art biosensor, and further, of analytical system and method of measuring even extremely small amount of sample with high response ability and high sensitivity by using micro miniaturized biologically active substance immobilized electrode.

The inventors have found that a biologically active substance(s) such as enzyme and antibody can be immobilized in the interior or on the surface of conductive material by immersing the surface of porous conductive material consisting essentially of fine particles in a solution containing said biologically active substance(s) to be adhered, and then coating with polymeric film. Further, in accordance with the present invention, a micro bioelement having high sensitivity and molecular selectivity provided with a surface layer incorporating said biologically active substance(s) immobilized can be obtained by dipping the fine conductive porous layer formed on the surface of an electrode in a solution containing the biologically active substance(s) to be incorporated therein, and then, coating with a polymeric film.

In the prior art flow injection analysis and batch form analysis, the inventive novel micro bioelement being used as a functional electrode in a flow cell can provide a quick and highly sensitive flow injection analysis or a batch form analysis. Further, the apparatus and method according to the invention overcome the difficulty of the prior art biosensor.

The inventive micro biosensor has surface area of several thousands time of the apparent surface area because of its fine particles of the surface layer of the micro electrode, and then, has high selectivity ability, and further very high response rate but generates output with high S/N ratio, and then, can improve the detection sensitivity when it is used as a functional electrode in a flow cell or batch cell. The inventive biological analysis has a high resolution in the wide range of the concentration of the target.

Because the electrode surface of this invention is in very fine particles, a sufficient amount of the biologically active subtance(s) can permeate deeply in the electrode. It has further the biologically active subtance(s) directly immobilized therein to enable to give high resolution and stable measurement and further high sensing maintenance. The electrode consisting essentially of platinum black can be in various form such as in disc form, in spherical form or in tubular form. Therefore, it can satisfy any requirements based on measurement by biosensor, other conditions, and then, it can be utilized for bioreactor. The inventive micro bioelement is extremely small and then the amount of samples can be very small, and further, the whole size of the analytical apparatus can be minimized, and can provide ability of measurement rate of so hundreds per one hour. Further, the inventive element can be assembled in array, and give a multiple functions to easily measure multiple components at the same time in bioanalysis. The inventive analytical apparatus can use a potentiometry.

The microelectrode having a biofunction fabricated in accordance with the present invention has a layer of fine particles of electrically conductive material consisting essentially of platinum and the like, which incorporates the bioactive substance such as enzyme, as formed on the surface of very small plate electrode (e.g. size of 1 to 500 micrometer ). Particularly, the electrode having a surface layer of platinum black formed by electrochemical deposition of platinum is well known to evidence high catalytical activity for hydrogenation, but the incorporation of the biologically active substance in such platinum black has not been known, While there has been known immobilization of enzyme and the like in the pores of platinum plate fabricated by etching, and then binding the enzyme thereon by a crosslinking agent, those porous members has only several m²/g of the surface area in ordinary condition. Further, it has not been known that the size of the platinum black can be controlled as in accordance with the present invention so as to incorporate the biologically active substance therein, to immobilize thereby the biologically active substance. An optional coating polymeric film in the inventive electrode functions to prevent completely dissolution of the enzyme, and to give biological adaptation. The enzyme being crosslinked in the fine conductive particles can not be dissolved so as to give a biologically adapted electrode.

In accordance with the present invention, biologically active substance is immobilized or unified to the fine particles of an electrically conductive material so as to fabricate a surface layer of the fine particles of an electrically conductive material incorporating the biologically active substance, by immersing the metal electrode having the conductive particulate layer in an aqueous solution of the biologically active substance(s), and then, impregnating with polymeric material such as albumin or heparin so as to prevent trace dissolution of the biologically active substance(s) and to give anti-thrombus ability and then, insolubilizing by a crosslinking agent so as to form an insoluble polymeric film thereon. Therefore, the inventive method of immobilizing can be said to be application of method of whole immobilization of enzyme in a gel or polymer, that is, the whole inclusion method to a metal electrode. The film being formed is several thousands Angstrom or less thick as finished, and then, the presence of the film cannot affect the activity of the biologically active substance(s) immobilized.

Hereinafter, "biologically active substance" may include enzyme and antibody as a representative, microorganism, organelle, antigen, antibody and hapten. Further, in the present invention, in place of platinum, may be "an electrically conductive material" such as gold, rhodium, palladium, and iridium used.

A polymer material used for covering additionally the surface of the biosensor of the present invention may include protein such as albumin, polysaccharide such as heparin. The usable crosslinking agent is preferably a crosslinking agent adapted to the used polymer material which may include glutaraldehyde adapted to albumin, and further include carbo diimido, maleate imide crosslinking agent.

The micro bioelement fabricated by the invention may include a bioelectrode for measurement and a transducer or a bioreactor in use for a reaction catalyst. The inventive element can be used for so various applications, and may be assembled in an apparatus for such applications.

The inventive method of immobilizing the biologically active substance is one of extremely important technology for development of clinical chemical analysis, and portable health check system requiring multiple detecting and multiple functions. Recently, there have been proposed various multiple biosensor using integrated circuit technique, and the inventive method of immobilization of enzyme and the like on a very small surface of the electrode is important in this phase too. Further, it is evident that the enzyme electrode fabricated by the inventive method has high sensitivity and highly quick response.

Further, mediater such as ferrocene can be incorporated in the fine particles of the biologically active substance so as to enable measurement of target material even in absence of soluble oxygen or with less soluble oxygen where oxygen has to be dissolved in the solution containing target material for the measurement. Further it enables to reduce significantly the potential necessary to operate the sensor.

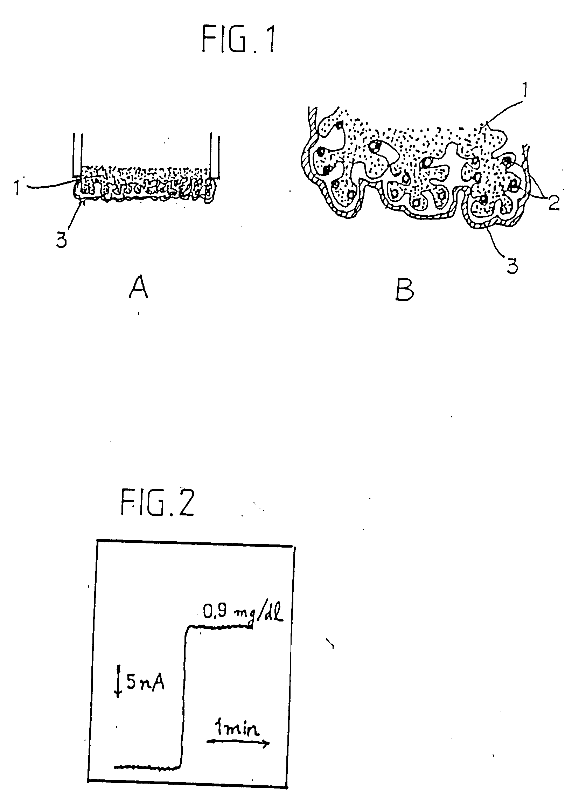

The structure of the electrically conductive material layer (of the inventive micro bioelement) incorporating the immobilized biologically active substance is as shown in FIG. 1 A, B. The fine particles of the the biologically active substance 2 are incorporated homogeneously as shown in the conductive fine particles.1. For example, electrically deposited platinum particles formed on the surface of the substrate is bulky and black precipitation, and the deposited platinum black can not be easily peeled even by agitating the aqueous solution, and is incorporating the biologically active substance(s) such as enzyme. Further, the platinum black layer is impregnated with polymer such as albumin, and treated with crosslinking agent such as glutaraldehyde so as to prevent dissolution of the biologically active substance(s).

In accordance with the present invention, the biologically active substance is immobilized in high density in a conductive material so as to afford a highly sensitive electrode in use for biosensor. For example, the electrode having an immobilized enzyme and the like in a surface layer of platinum black of a platinum electrode has a high sensitivity when it is used as a biosensor in use for measurement by amperemetry. Therefore, using a method of immobilizing the biologically active substance in accordance with the present invention, a biosensing system can be fabricated using the microelectrodes.

The enzyme and the like are incorporated in a small size electrode by electrolysis depositing the conductive material in very fine particle on the surface of the electrode, so as to form a biologically active substance(s) immobilized electrode. The size of fine particles of the biologically active substance or the distance between the particles can be adjusted by changing the preparation conditions.

As a material for electrode, gold, other noble metal, carbon such as glassy carbon, graphite carbon as well as platinum can be used for a substrate, and then, fine particulate material such as rhodium fine particles, gold fine particles, platinum black, carbon, fine particles of graphite, or fine particles of conductive metal oxide, ruthenium oxide (RuO₃), palladium, iridium and the like in layer form is formed on the surface of the substrate, in such porous layer of which the biologically active substance(s) is immobilized.

The microelectrode in accordance with the present invention having a surface layer of fine particles of electrically conductive material such as platinum including the biologically active substance such as enzyme is used as a functional electrode in a flow injection measurement or batch measurement, and can be assembled in a flow cell or batch cell with a reference electrode and a counter electrode. In accordance with the present invention, the active substance(s) immobilized microelement is used in a flow cell or a batch cell for bioanalysis by applying a potential so as to measure the generated current on the electrode, in which the concentration of trace in the solution in the cell. Therefore, it can provide easy and uncostly fabrication of the bioanalytical measurement apparatus.

The method of fabricating the microelectrode used for the present invention may comprise immersing fine conductive particles material in a solution containing the biologically active substance(s) to impregnate the biologically active substance deeply in the conductive particulate material, or adhering the biologically active substance(s) on the surface thereof, and then, crosslinking directly the biologically active substance(s) one the other, or forming polymeric film on the surface of the conductive particles to be covered so as to prevent the dissolution of the biologically active substance(s). That is, the fabrication can be accomplished by the two steps. Such fabricated bioelectrode can be in a very small size, and functions as a biologically active element, by having the structure of surface layer of fine particles incorporating the biologically active substance(s). Such electrode is referred to as a microelectrode or a microelement. The biologically active substance(s) immobilized electrode can be used as a functional electrode in a flow injection measurement or a batch measurement by applying a constant potential to generate sensitively current responding to the trace material biologically reactive, and then, the concentration of the trace can be determined with high speed, and high sensitivity.

Such utilization of platinum black preparation and enzyme electrochemical adsorption technique makes it successful to unify porous conductive material and the biologically active substance. The microelectrode has therefore highly quick response and high sensitivity.

The inventors developed further to make a bioanalytical system of measuring in a flow injection or batch form to determine the concentration of the trace in the sample.

The microelectrode has the structure of unifying the electrode and an enzyme molecular. That is, a platinum black particulate layer is not only a stationary carrier for an enzyme, but also a transducer. In the microelectrode of the present invention, a surface area of the transducer can be several hundreds time of the prior art transducer, and the inventive microelectrode can be classified into a micro in view of size, but can be said to be in macro form in view of the surface area being large, and giving a high S/N ratio in measuring.

Because the microelectrode has a unification of electrode and enzyme, hydrogen peroxide generated by application of a given potential can be effectively oxidized. Then, the micro electrode of the present invention was tested to evaluate its response ability by measuring in a buffer solution of phosphoric acid of a measurement apparatus. The result showed that the electrode can respond within three seconds showing quick response. It evidences that the biosensor of the present invention can measure even in order of the concentration of 0.1 mg/dl, and the linearity of the value and the concentration was found within the range to be measured of 0.1 mg/dl to 100 mg/dl.

The inventors invented further a bioanalytical apparatus and method by using the inventive microelectrode in a flow injection or batch measurement. The microelectrode is used as a functional electrode and assembled in a flow cell or a batch cell in which a liquid containing the trace to be measured is put in, or so as to measure the trace in a solution to be put in with very high speed.

The microelectrode having a biofunction fabricated in accordance with the present invention has a very small plate electrode (e.g. size of 1 to 500 micrometer ) of platinum and the like, which incorporates the bioactive substance such as enzyme, as formed on the surface of very small plate electrode. Particularly, the electrode having a surface layer of platinum black formed by electrochemical deposition of platinum is well known to evidence high catalytical activity for hydrogenation, but the incorporation of the biologically active substance in such platinum black has not been known (while there has been known immobilization of enzyme and the like in the pores of platinum plate fabricated by etching, and then binding the enzyme thereon by a crosslinking agent.) Further, it has not been known that the size of the platinum black can be controlled as in accordance with the present invention so as to incorporate the biologically active substance therein, to immobilize thereby the biologically active substance.

The method of fabricating the microelectrode used for the present invention may comprise two steps. For example, firstly, a layer of conductive fine particles such as platinum black is formed on the end surface of platinum wire (in general, electrically conductive material), and then, the active substance(s) is immobilized therein. Concretely, the electrode having a layer of conductive fine particles immersed in a solution containing the biologically active substance(s), or coated with the solution, so as to impregnate the biologically active substance deeply in the conductive particulate material, and then, further, impregnated with a polymeric material such as albumin or heparin, and then, crosslinking directly the biologically active substance(s) one the other, and then, impregnating with polymeric material such as albumin or heparin so as to prevent trace dissolution of the biologically active substance(s) and to give anti-thrombus ability and then, insolubilizing by a crosslinking agent so as to form an insoluble polymeric film thereon. forming polymeric film on the surface of the conductive particles to be covered so as to prevent the dissolution of the biologically active substance(s). Therefore, the inventive method of immobilizing can be said to be application of method of whole immobilization of enzyme in a gel or polymer, that is, the whole inclusion method to a metal electrode. The film being formed is several thousands Angstrom or less thick as finished, and then, the presence of the film cannot affect the activity of the biologically active substance(s) immobilized.

Accordingly, the size of the microelectrode is dependent on the diameter of the used platinum wire, and then, when a very thin wire is used, the bioelectrode can be significantly smaller than that expected in the prior art. Therefore, the bioelectrode with size in micron order can be easily fabricated, and can be conveniently used for medical application e.g. intravital application, and then when it is used for analytical purpose, an apparatus with very little sample cell can be manufactured so as to enable analysis of trace amount sample, and further, quick measurement and high response ability can be easily afforded. In addition, when the electrode with larger size is fabricated, the analytical apparatus can be operated with the higher output for detection.

The electrode consisting essentially of platinum black can be in various form such as in disc form, in spherical form or in tubular form. Therefore, it can satisfy any requirements based on measurement by biosensor, other conditions, and then, it can be in appropriate form.

The conductive material to be used as a substrate can be gold, other noble metal, as well as carbon, grassy carbon, graphite in place of platinum. Then, platinum black, gold black, noble fine particles, and conductive metal oxide in a particulate layer can be used with the active substance.

In the fabrication of the inventive microelectrode, the conductive material to be used as a substrate should not necessarily be the same as the conductive material to be deposited as fine particles (porous material). For example, the structure in which platinum black is deposited on the surface of graphite can be used. Further, gold and rhodium, palladium and iridium can be used in place of platinum for the formation of porous conductive material.

The microelectrode used in the present invention can be covered with polymeric material such as protein and polysaccharide for coating, and then, crosslinked with the cover film by crosslinking agent so as to give a biological adaptation, to have longer life, and to minimize the resolution of the biologically active substance.

"Biologically active substance" may include enzyme and antibody as a representative, microorganism, organelle, antigen, antibody and hapten.

A polymer material used for covering additionally the surface of the biosensor of the present invention may include protein such as albumin, ion exchange resin, polysaccharide such as heparin. The usable crosslinking agent is preferably a crosslinking agent adapted to the used polymer material which may include glutaraldehyde adapted to albumin, and further include cardo diimido, maleate imide crosslinking agent.

The method of depositing porous (particulate) conductive material for preparing the inventive microelectrode can use non-electrolysis whereas the above mentioned fabrication is carried out by electrolysis deposition.

Further, the micro enzyme electrode is electrochemically treated or anodizingly treated so as to improve the selectivity on the measurement. Those treatments can attenuate the non-unique activity of the platinum and the like, thereby to evidence the improved activity merely of the enzyme.

The inventive analytical method can practice a miniatured bio sensing system with high speed response and high sensitivity. This will be extremely important technique for the development of clinical analysis or portable health checking system requiring miniaturization of biosensor and multiple detecting or multiple function.

The inventive method of immobilizing the biologically active substance can afford the inventive bioelement (electrode), which will provide firstly highly sensitive detection, and secondly a whole incorporation of the biologically active substance(s) such as enzyme without any harm to the active substance(s) by easy and direct immobilization. Thirdly, it will provide a high density of the active substance(s) immobilized on the surface of the microelectrode to afford very quick response. and fourthly, an easily and readily immobilization of the active substance(s) such as enzyme without any chemical treatment on the very small surface of the electrode, and fifthly, a multifunctional enzyme sensor with a very small surface, in which plurality of enzymes are immobilized or settled. The inventive electrode element can measure quickly and sensitively because it has an apparent small surface area, but, a very large surface area several thousands times than the apparent surface area of the electrode which is fabricated from the deposition of fine particles on the surface of the electrode, so as to increase the sensitivity of the element.

An analytical system or method in accordance with the present invention can provide firstly a high performance biosensing system with highly quick response and high sensitivity which enables flow measure, and secondly provide a bioanalytical system with high resolution and stability, because the microelectrode is incorporating stably and wholly the active substance(s) therein. Thirdly, it provide an extremely good flow injection analytical apparatus because of the microeletrode impregnating the active substance stably immobilized therein. Fourthly, it enables to evidence the linearity in the wide range of 50 micro Mole to 50 milli Mole of the glucose concentration. Fifthly, it uses a platinum black preparation, and then, can be easily fabricated in any form of various forms such as disc, spherical, or tubular, and therefore, can be used in means for biologically functioning. Sixthly, it will provide a biological analytical apparatus with high performance of enzyme sensor and high stability. Because the microelectrode can be very small, the sample can be measured even in extremely small amount, and further, the total apparatus can be miniaturized. Further, very high speed measurement such as several hundred samples per one hour can be easily treated in a continuous measurement, and the electrode can be assembled in array, and facilitates multifunctional measurement to measure multiple components in the sample at the same time in flow analysis or batch analysis.

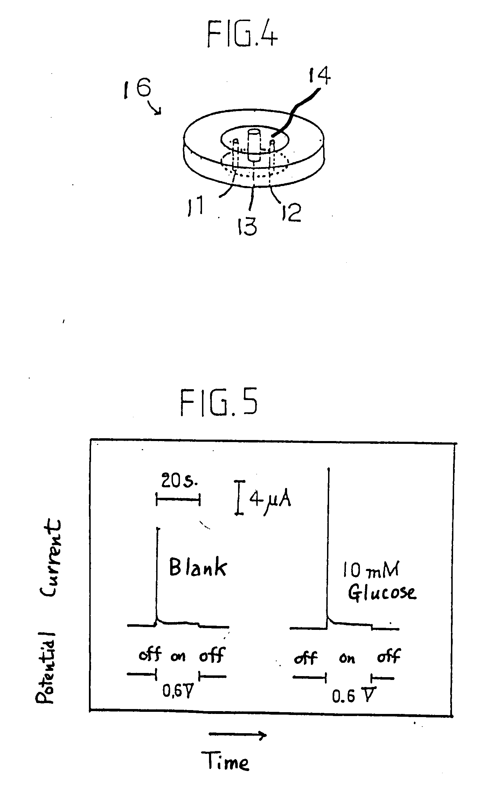

FIG. 4 shows a micro device 16 comprising micro electrode 11 with diameter of about 1 micrometer to 500 micrometer as a functional electrode, a counter electrode 12 of platinum wire, and a reference electrode 13 of silver/silver chloride, those three electrodes fixed in the resin14 settled in a hole of PTFE (poly tetrafluoro ethylene) resin. Since such micro device 16 comprises merely three metal wires as settled therein, the device can be in very small size.

When such micro device is used, it enables to measure on sample in very small amount, for example, solution in merely one micron liter. After titration of very small amount sample, potential is applied, and the generated current thereby is detected to determine the amount of the target material in the sample.

Accordingly, the active material as generated in the electrode by the biologically active substance such as enzyme molecular can be directly measured in real time.

Various voltammetric measurement can be applied to the above electrode, and then, the sample can be measured on even in a stationary state.

The inventive bioanalytical system will detect active substances within the electrode, and then oxidizing enzyme and dehydrogenating enzyme can be immobilized.

The inventive method of immobilizing the biologically active substance can afford the inventive bioelement (electrode), which will provide firstly a good response ability, and highly sensitive detection, and secondly a whole incorporation of the biologically active substance(s) such as enzyme without any harm to the active substance(s) by easy and direct immobilization. Thirdly, it will provide a high density of the active substance(s) immobilized on the surface of the microelectrode to afford very quick response, and fourthly, an easily and readily immobilization of the active substance(s) such as enzyme without any chemical treatment on the very small surface of the electrode, and fifthly, a multifunctional enzyme sensor with a very small surface, in which plurality of enzymes are immobilized or settled. The inventive electrode element can measure quickly and sensitively because it has an apparent small surface area, but, a very large surface area several thousands times than the apparent surface area of the electrode which is fabricated from the deposition of fine particles on the surface of the electrode, so as to increase the sensitivity of the element.

An analytical system or method in accordance with the present invention can provide firstly a high performance biosensing system with highly quick response and high sensitivity which enables to measure on stationary sample, and secondly can enable to reduce the amount of the sample to be measured, because micro enzyme electrode ( i.e. microelectrode), counter electrode and reference electrode can be arranged in a very small surface. Thirdly, it can detect directly an active material generated by enzyme moleculars within the electrode so as to measure in real time. Fourthly, various voltammetry technique can be used so as to enable detecting with enough sensitivity even in a stationary state. Fifthly, it uses an active substance(s) in the electrode, and then, important enzyme or biologically active substance(s) such as oxidizing enzyme and dehydrogenating enzyme can be used. Sixthly, it will provide an intravital biosensing system or portable biosensor.

The analytical apparatus can use a potentiometry for measurement. Further, it can be developed to sensing device to be assembled in a system.

### SIMPLE DESCRIPTION OF DRAWINGS

FIG. 1 A illustrates schematically the section of structure of the bio element prepared by the inventive method of immobilizing the biologically active substance. FIG. 1 B illustrates schematically the enlarged section of a portion of FIG. 1 A, as shown by a dotted line, to show the detailed structure of fine particulate conductive material impregnated with the biologically active substance.

FIG. 2 is a graph showing the response ability by the biosensor element in the batch system measurement.

FIG. 3 is a graph showing the relationship of responded sensor output and glucose concentration measured by the biosensor element in the batch system measurement.

FIG. 4 illustrates schematically the perspective view of micro device of three electrode system( measuring by pulse method), using the biosensor element as shown in FIGS. 1 A and 1 B of the present invention.

FIG. 5 is a graph showing the current generated when a constant pulsed potential is applied to the working electrode of the micro device of FIG. 4 and the resulting transient current response recorded by a strip chart recorder.

FIG. 6 is a graph showing the relationship of generated current value and glucose concentration measured by the biosensor element as shown in FIG. 5 of the present invention.

FIG. 7 is a graph showing the relationship of the volume of glucose sample and peak current value as measured by the analytical system in accordance with the inventive pulse method.

FIG. 8 is a graph showing a transient current response to a pulsed potential applied to the working electrode of the micro device of Fig. 4, where the data are recorded by a transient memory.

FIG. 9 is a graph showing the relationship of the glucose concentrations and the difference between the response current and the blank response current as measured at two millisecond after the application of the pulsed potential shown in FIG. 8.

FIG. 10 is a graph showing the relationship of the glucose sample quantity and the difference of the current value as measured after two milliseconds after the application of the pulsed potential shown in FIG. 8.

FIG 11. illustrates schematically the structure of the detecting portion of the flow injection analysis using the bio element of the present invention.

FIG. 12 is a graph showing the result in its response ability when the sample containing glucose is measured by using the flow injection analytical apparatus as shown in FIG. 11.

FIG. 13 is a graph showing the relationship of the glucose concentration and the response output, as measured by using the flow injection analysis in accordance with the present invention.

FIG. 14 is a graph showing the improvement in the selectivity of the trace substance to be detected, as measured by using the oxidation treated bio electrode element in accordance with the present invention.

FIG. 15 is a graph showing the selectivity of the trace substance to be detected, as measured by using the oxidation not- treated bioelectrode element.

### BEST MODE FOR CARRYING OUT THE INVENTION

The structure of the bio element, as fabricated by the inventive immobilization of the biologically active substance in accordance with the present invention in shown in FIGS. 1 A and 1 B in the sectional view and its enlarged view. There is provided micro electrode having the fine particles 1 of the electrically conductive material, the biologically active substance 2, and the polymeric film 3.

FIG. 4 shows micro device 16 comprising a micro enzyme (glucose oxidase) device 16 having a micro electrode 11 of about 1 micrometer to 500 micrometer in diameter, as a functional electrode, and a counter electrode 12 of platinum wire, and a reference electrode 13 having silver/silver chloride. Those three electrodes, i.e. microelectrode 11, the counter electrode 12 and the reference electrode 13 are fixed in the resin 14 put in the hole of PTFE (poly tetrafluoro ethylene ) and settled. Such micro device 16 has the structure having merely three metal wire fixed, and therefor, can become very small.

Therefore, the micro electrode can detect on a very small quantity of sample, for example, one microliter of the sample. After the very small amount of sample is put in, the potential is applied, and then the generated current value is detected to determine the amount of the trace substance to be detected, in the sample.

Accordingly, the inventive device can detect directly the active substance generated in the inventive electrode by the biologically active substance for example enzyme contained in the micro electrode. Further, the inventive electrode can be exerted by voltanemetery detecting method in various form, and then, the sample can be detected even in a stationary condition.

Because the inventive electrode can detect the active substance therein, it can exerted for the bioanalysis of the important biologically active substance such as oxidized enzyme or dehydrogenated enzyme.

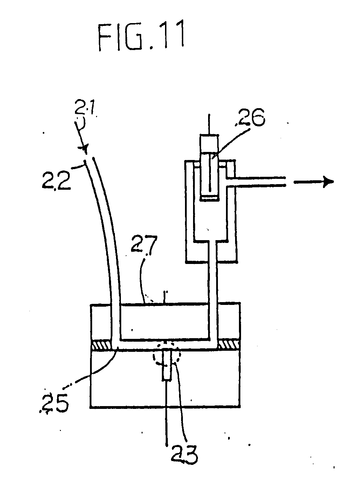

FIG. 11 illustrates in sectional view the analytical apparatus by using flow injection method in accordance with the present invention. The liquid 21 containing the trace substance to be detected is charged from an inlet 22, passes through a tube having a micro electrode 23, and passes through a container having a reference electrode 26 and a counter electrode 27 so as to detect the trace substance contained in the liquid sample 21. The micro electrode 23 of the present invention has a unified structure of the biologically active substance and platinum black on the face of the platinum wire, and then, the device can be fabricated in very small size, for example, the size of micrometer order in diameter can be microfabricated, and then the flow cell as shown in the drawings can be fabricated in very small size.

Accordingly, the bio sensing system of the present invention is miniaturized so as to detect the very small quantity even in a small amount of the sample, when the biologically active substance immobilized electrode, for example, enzyme embodied electrode is utilized. Further, in the continuous measurement, several hundreds of samples can be treated per one minute of time.

Further, the bio analytical system of the present invention can use a potentiometry measurement.

The present invention is detailedly illustrated by the following examples, but should not be interpreted for the limitation of the invention.

### EXAMPLE 1

### Fabrication of biologically active substance immobilized electrode and its feature measurement

Glucose is changed into gluconic acid and hydrogen peroxide in the presence of glucose oxidase, and then, its concentration can be determined by measuring the oxidation current due to hydrogen peroxide responding to the glucose concentration by using an electrode incorporating the enzyme with platinum electrode. In this regard, the preparation of enzyme fixed electrode would be illustrated, and then, the test for measuring glucose concentration by using the resulting electrode was carried out.

A platinum wire of 100 micrometer in diameter was fixed at a tube of soda glass and was polished at its end surface by alumina powder to form a very small surface of a platinum electrode. Application of potential was carried out for 30 minutes in a 0.5 M solution of sodium sulfate, using silver/silver chloride electrode as a reference electrode, the potential of the platinum electrode being + 1.3 V to -0.25 V, and the scanning rate being 100 mV /second. Electrolysis was carried out for 10 minutes in a solution of 3 % hexachloroplatinate containing 300 ppm of lead acetate at the current of - 50 microampere to precipitate platinum black. The precipitated platinum black layer was about several micrometer thick. Then, the resulting platinum black electrode was dried at 25 °C for 60 seconds by blowing air. Then, 0.3 V was applied to the resulting platinum black electrode being a functional electrode, for 30 minutes by using silver/silver chloride electrode as a reference electrode so as to generate hydrogen gas from the platinum black electrode.

After the resulting platinum black electrode was dried at 20 °C for 60 seconds by blowing, it was immersed for 20 minutes in 1 ml of a phosphoric acid buffer solution (pH=6.8) containing 5,500 units of glucose oxidase. Then, the electrode was for 10 minutes in 1 mililiter of phosphoric buffer solution (pH=6.8) containing 10 % of albumin, and further, immersed for 10 minutes in one mililiter of phosphoric buffer solution containing 2 % of glutaraldehyde. The resulting electrode was washed overnight in a 0.1 M buffer solution of phosphoric acid.

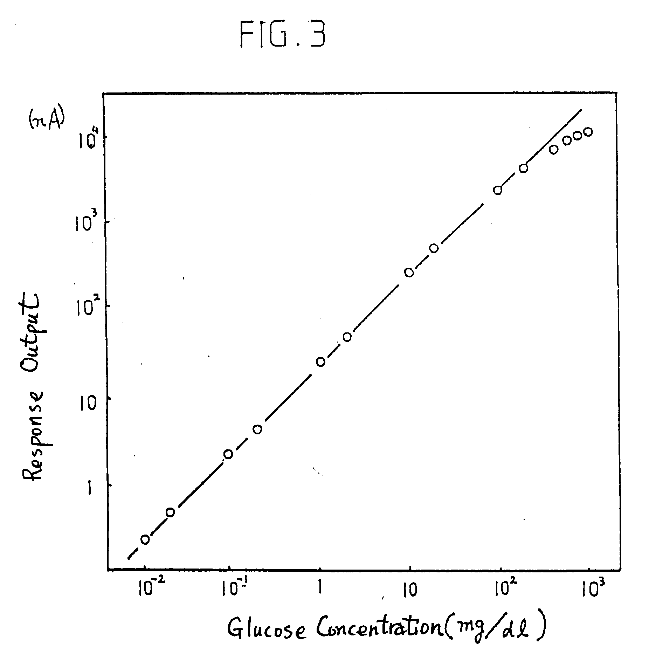

The bio sensor electrode of the present invention was tested to evaluate its response ability by measuring in a buffer solution of phosphoric acid of a measurement apparatus comprising a reference electrode (standard electrode ), a counter electrode (platinum electrode and a functional electrode i.e. the glucose oxidase fixed platinum black electrode prepared in accordance with the present invention). Each of those electrodes were connected respectively to the each electrodes of potentiometeric apparatus. Plus 0.6 volt was applied to the functional electrode relatively to the reference electrode, and glucose was added to, and the oxidation current generated by hydrogen peroxide between the counter electrode and the functional electrode was measured. The result showed that the electrode can respond within three seconds to 100 %. As shown in FIG. 3, it evidences that the biosensor of the present invention can measure even in order of the concentration of 0.1 mg/dl, and the linearity of the value and the concentration was found within the range to be measured of 0.1 mg/dl to 100 mg/dl.

The output of the sensor of the present invention associated with the addition of glucose to the solution (0.9 mg/dl), that is, the change of the oxidation current generated by hydrogen peroxide shows a very quick response as shown in FIG. 3. It evidenced to respond within three seconds to the rate of 100 %. The response of the sensor was very quick, and reached rapidly to the constant value. It was apparent that the biosensor prepared by the immobilization of the biologically active substance in accordance with the present invention has a quick response ability and high sensitivity, which can be easily and readily fabricated. It is evident that the prepared electrode by the inventive method of immobilizing the bioactive substance can easily be fabricated, and has quick response and high sensitivity and further can used for the fabrication of micro biosensor by a simple method.

### Example 2

### Analytical apparatus and the method thereby using the micro electrode in the pulse method.

A fine platinum wire of 100 micrometer in diameter, a platinum wire of 200 micrometer in diameter for a counter electrode, and a silver wire of 500 micrometer in diameter were fixed by the resin 14, and then, the surface of the fine platinum wire was polished by using alumina powder. Against such polished surface, enzyme was immobilized as follows.

The polished wire was dipped in the 3 % solution of platinumic chloride,containing 300 ppm of lead acetate, and electrolysis was exerted for ten minutes, by applying a constant current of - 50 microampere, to form a deposit of platinum black of about several micrometer. After the resulting platinum black electrode was dried at 25 °C for 60 seconds by blowing, the electrode was maintained at minus 0.3 V for 30 minutes in a 0.5 M sulfate aqueous solution so as to generate hydrogen gas from the platinum black. Then, it was further dried at 20 °C for 60 seconds by blowing, and then, was immersed for 20 minutes in one milliliter of phosphoric buffer solution (pH=6.8) containing glucose oxidase, and, dried again by blowing.

Then, the micro device 16 using the micro electrode as shown in FIG. 4, was obtained after a silver wire was firmed into a silver/silver chloride reference electrode, washed in a 0.1 M buffer solution of phosphoric acid overnight resulting in three electrode microdevice. FIG. 4 shows a functional electrode ( microelectrode ) 11, a counter electrode 12, a reference electrode 13, resin 14, PTFE ( poly tetrafluro ethylene ) and the microdevice 16.

### Measurement of glucose concentration by using enzyme immobilized electrode of the present invention.

20 Microliter of glucose sample was put in the micro device of three electrodes system as shown in FIG. 4, and the potential of 0.6 volt was applied. That is, several samples of different concentration of glucose were dropped in the device applying 0.6 volt of potential. The peak current values generated were measured and the relation of the measured current values and the glucose concentrations was investigated. The result is as shown in FIG. 5.

When 0.6 volt of potential was applied, the current as shown in FIG. 5 was generated. Where the sample did not contain glucose, the current as shown the left portion of FIG. 5 was generated. Where the sample was a buffer phosphoric acid solution containing 10 mM glucose ( 50 mM, pH=7, 0.05 mM NaCl ), the response current as shown in the right portion of FIG. 5.

As apparent from FIG. 5, the response current was generated immediately after the potential was applied, and then, the response current was rapidly reduced. This will support that the hydrogen peroxide generated by glucose oxidizing enzyme would be oxidized directly and immediately when the potential was applied. Further, it was understood that when the potential was repeatedly applied, the peak current will be at a constant value after several time cycle of application.

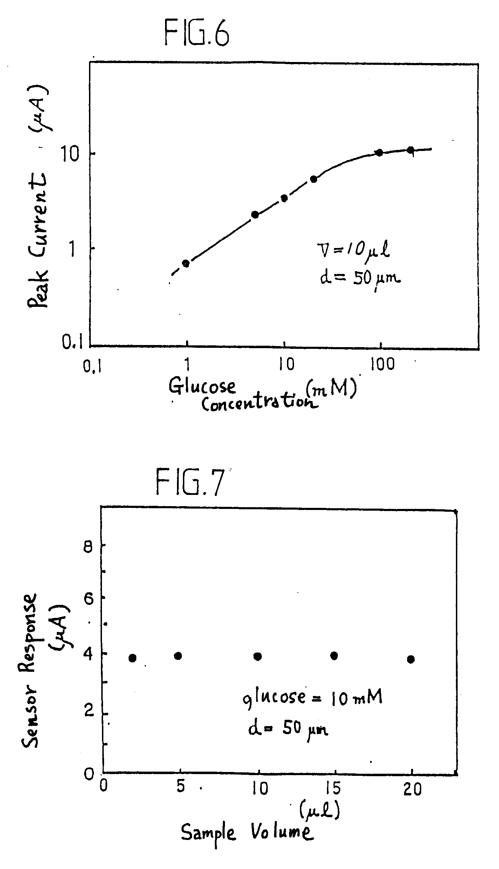

Such phenomenon was found when the potential was applied in a buffer solution of phosphoric acid in the similar way. Then, the peak current values were measured to the samples containing the given concentration of glucose, and the peak current generated in a buffer solution not containing glucose was measured ( blank value ). The difference values from the peak value to the peak value at not -containing glucose solution were calculated, and the relation of the difference values and the given glucose concentrations was investigated. The result is shown in FIG. 6. That is, where the glucose concentration changes from 1 mM to 100 mM, the measured curve showing the relation to the peak current is found as in FIG. 6.

Therefore, it is supported that the enzyme analytical system in accordance with the present invention can measure the concentration of the trace substance in real time even in the stationary sample in a very small amount.

### Test of sample quantity dependence as measured by the device of the present invention.

2, 5, 10, 15, and 20 microliter of the glucose standard sample containing 10 mM of glucose were respectively picked up for each tests and dropped in the micro biosensor of the present invention, and the currents generated when the potential of 0 .6 volt was applied were measured for each test. The result is shown in FIG. 7. It was found that the peak current generated is independent to the quantity of the sample.

### Selective measurement of concentration by Transient response measurement in pulse voltammetry

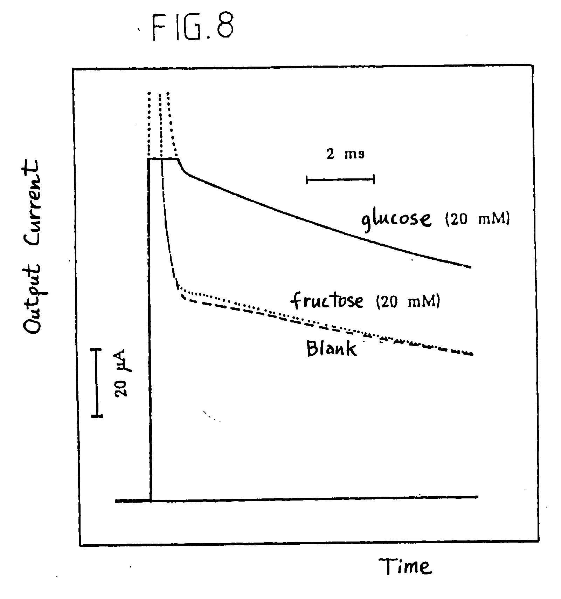

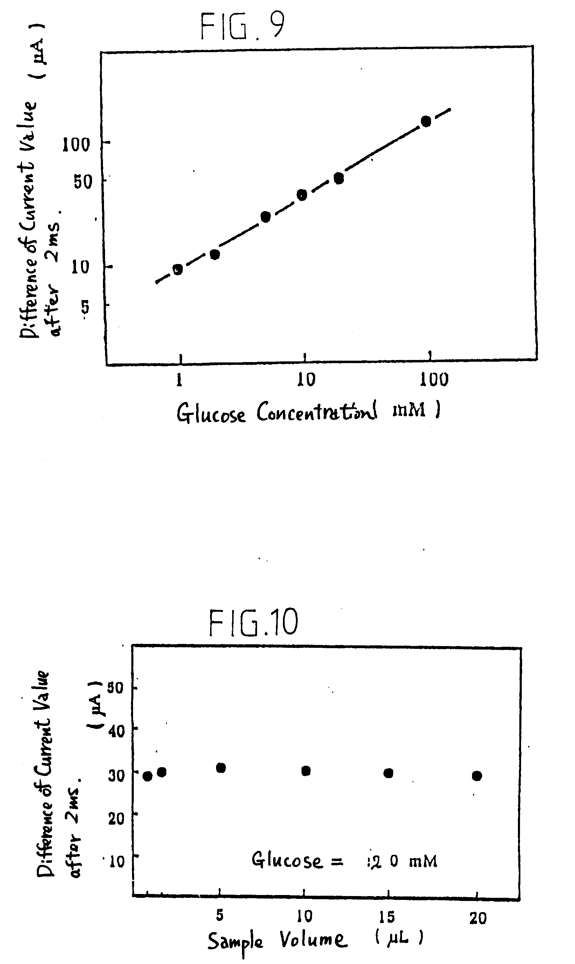

The initial stage of the response of the sensor was recorded by a transient memory when the potential of 0.6 volt was applied to the enzyme electrode for the detailed measurement of the pulse shown in the right portion of FIG. 5. The result is sown FIG. 8. There is found a significant difference of the initial response to the glucose containing buffer solution from the initial response to the blank sample ( phosphoric acid buffer ) and to the fructose-containing phosphoric acid buffer. Therefore, The difference of the the current value at two microseconds time after the application of the potential from the glucose containing sample to the blank sample is plotted against the concentration of glucose as shown in FIG. 9. The linearity of the output to the concentration of glucose was found in the range of 1 mM to 100 mM.

Further, the relation of the sample amount to the output of the sensor is investigated where the concentration of glucose is constant. The measured result is shown in FIG. 10. It was confirmed that the output of the sensor is not dependent on the amount of the samples.

It is noted that the analytical system and method in accordance with the present invention can give information on the whole sample under stationary state by detecting on a portion of the sample in the measurement.

Further, the analytical system and method in accordance with the present invention does not need the agitation of the sample, and therefore, can be used as an intravital biosensing system or as a portable biosensor.

The bioanalytical system of the present invention can be used in a countercurrent vessel or in a batch form as well as in a stationary vessel, and can detect with high speed response and quick measurement and high sensitivity.

### Example 3

### Analytical apparatus and method by flow injection using the micro electrode of the present invention

There will be illustrated a method of fabricating enzyme embodied electrode ( micro electrode ) to be used for the inventive analysis. First of all, 6 species of platinum wires having the different diameter of 10 micrometer to 3,000 micrometer were picked up. the end surfaces of those small platinum wire were polished by alumina powder to be a micro platinum electrode. Application of potential was carried out for 30 minutes in a 0.5 M aqueous solution of sodium sulfate, using silver/silver chloride electrode as a reference electrode, the potential of the platinum electrode being + 1.3 V to - 0.25 V, and the scanning rate being 100 mV /second. Electrolysis was carried out for 10 minutes in a solution of 3 % hexachloroplatinate containing 300 ppm of lead acetate at the current of -50 microampere to precipitate platinum black. The precipitated platinum black layer was about several micrometer thick. Then, 0.3 V was applied to the resulting platinum black electrode being a functional electrode, for 30 minutes by using silver/silver chloride electrode as a reference electrode so as to generate hydrogen gas from the platinum black electrode.

After the resulting platinum black electrode was dried at 20 °C for 60 seconds by blowing, it was immersed for 20 minutes in 1 ml of a phosphoric acid buffer solution (pH=6.8) containing 5,500 units of glucose oxidase. Then, the electrode was for 10 minutes in 1 mililiter of phosphoric buffer solution (pH=6.8) containing 10 % of albumin, and further, immersed for 10 minutes in one mililiter of phosphoric buffer solution containing 2 % of glutaraldehyde. The resulting electrode was washed overnight in a 0.1 M buffer solution of phosphoric acid, so as to afford a microelectrode.

The bio sensor electrode of the present invention was used to fabricate the micro device as shown in FIG. 11. In a flow injection measurement, the liquid 21 to be measured flows from an inlet 22, and passes the passage 25 having a microelectrode 23. A silver/silver chloride electrode was used as a reference electrode 26, and a stainless steel block electrode was used as a counter electrode 27.

### Measurement of glucose concentration

A liquid to be measured was put into a flow injection measurement system as shown in FIG. 11 by a single pump ( ER-8711 type) manufactured by Erma Inc. 6 Species of micro electrodes were used having the diameter of 10 micrometer to 3 millimeter. A stainless steel block electrode was used as a counter electrode, and a silver/silver chloride electrode was used as a reference electrode. Potential of 0.6 volt was applied to the microelectrode, and hydrogen peroxide being generated was oxidized. 0.1 M phosphoric acid buffer solution ( pH=6.8 ) was used as a mobile phase, and several kinds of glucose containing samples having different concentration in 5 micrometer were put in. The flow rate varied in the range of 0.4 ml/minute to 1.8 ml/minute.

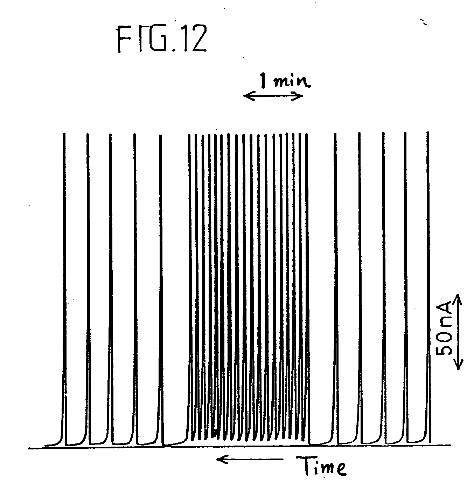

Using a microelectrode having a deposit of glucose oxidase incorporated platinum black formed on the end surface of a platinum wire of 100 micrometer in diameter, the response to the intermittent charge of glucose solution ( 10 mM ) was measured and recorded. The result is shown in FIG. 12. As shown in the drawings, the current generated reached at peak three seconds after charge of the sample, and returned to the original level within ten seconds. While nine samples were charged one after another within one minute, the detected current shows complete independence of the each peak corresponding to each charge of the samples. It was found that one sample could be detected within about nine seconds. The time period necessary for one sample to complete the measurement, that is, the response time for measurement of glucose was measured as a response time. The result is shown in Table 1.

It is apparent from the table that the time period necessary to measure is closely dependent on the diameter of the microelectrode. The response rates to measure the concentration of glucose were investigated changing the diameter of the platinum wire from 10 micrometer to 3 millimeter to form a platinum black incorporating glucose oxidase. The result is shown in Table 1.

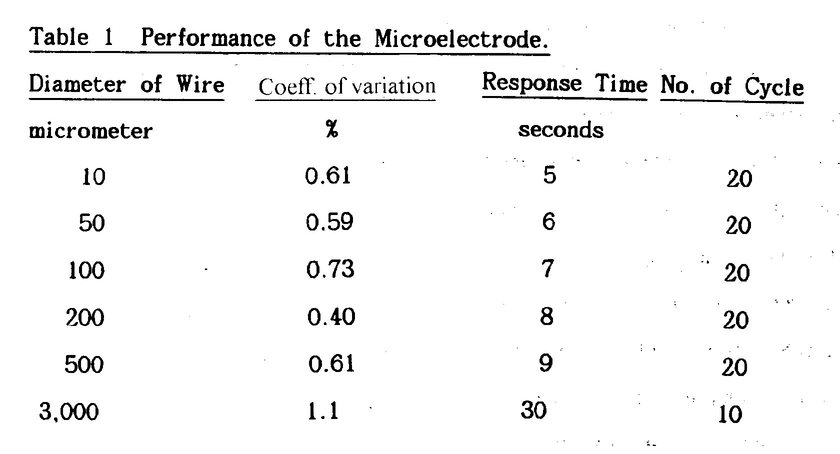

It is apparent from Table 1 that the time necessary to measure the concentration of glucose was shorter, when the flow rate was higher, and that the value of the peak current was lower, when the flow rate was higher. For example, while the microelectrode of 500 micrometer in diameter needs 10 seconds for the time to measure on glucose concentration, the microelectrode of 10 micrometer in diameter reduced significantly to 5 seconds for the measurement. This suggests that the decrease of the diameter of the microelectrode enables to measure in high speed.

Next, the time period necessary to measure the concentration of glucose and the peak current were investigated changing the flow rate of the mobile phase solution. As a result, it was found that the time necessary to measure the concentration of glucose was shorter, when the flow rate was higher, and that the value of the peak current was lower, when the flow rate was higher.

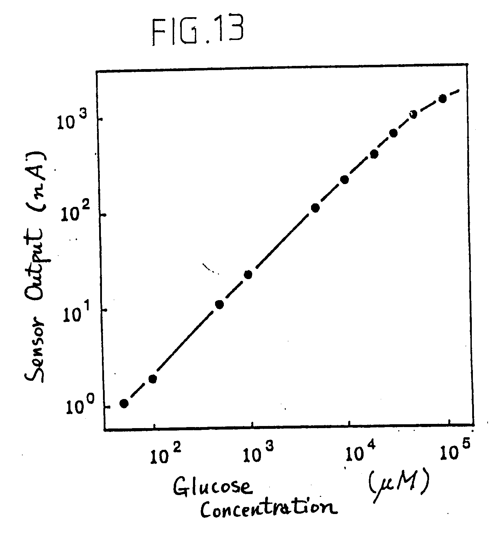

The relation of the glucose concentration and the peak current is shown in FIG. 13. It was apparent from FIG. 13 that the linearity of the peak current and the concentration of glucose is established in the range of 50 µ Mole to 20 mM.

### Test of Stability and Maintenance of the inventive analytical system.

The microelectrode as fabricated above was used repeatedly for the measurement. While about 600 samples were continuously put in the electrode cell, it was found that the peak current at the initial stage was the same as that at 600th sample, i.e. at one hour later. It was supported that a analytical system of the present invention could be operated stably, and the output of the inventive electrode was stable to the glucose.

Next, the coefficients of variation were calculated from the measured value of glucose concentrations. The result is shown in Table 1. It is found that except of the diameter of 3 mm, the change rate is within one %, and the measurement by the inventive electrode has extremely excellent accuracy.

Further, the stability of the inventive flow injection analytical apparatus was investigated for one month for long-period accuracy, and no lowering of the peak current value was found even after one month from the beginning.

### Example 4

### Result of anodic polarization

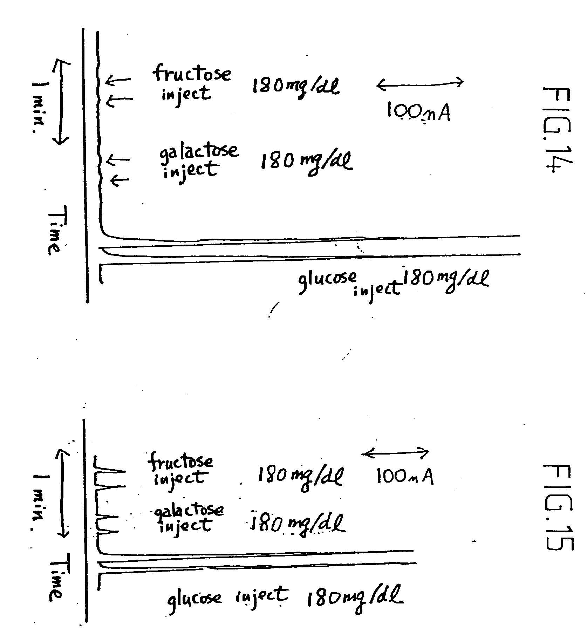

A platinum wire of 100 micrometer in diameter was sealed on a block of acryl resin, and the surface of the block was polished by alumina abrasives having particles of 30 micrometer to 0.05 micrometer. Next, it was put in a solution of hexachloroplatinate of 3 % containing 500 ppm of lead acetate, and electrolysis was carried out therein to form a deposit of platinum fine particles on the smooth surface of the platinum wire. This electrolysis was for five minutes carried out at - 0.08 volt. Six platinized electrode were fabricated, and then, among them, three of them were treated by oxidation (anodically polarizing) treatment, and three of them were not treated. The treatment was carried out by dipping the resulting platinized electrode, and using this electrode as a functional electrode, a silver/silver chloride electrode as a reference electrode in the three electrode system, and applying 1.2 volt to said enzyme electrode for ten minutes. Next, glucose oxidase was immobilized all on the electrodes treated and not-treated. The immobilization was carried out by immersing for 10 minutes in one milliliter of a phosphoric buffer solution (pH=6.8) containing 5,500 unit of glucose oxidase. Further, the electrode was dipped for 10 minutes in a phosphoric buffer solution (pH=6.8) containing 2 % of glutaraldehyde. Then, the resulting glucose oxidase embodied electrode was washed overnight with 0.1 N phosphoric acid buffer solution. This glucose oxidase embodied electrode was assembled as a functional electrode in the flow measurement apparatus as shown in FIG. 11. The sample solutions containing various kinds of saccharide were put in the measurement system in flow injection as shown in FIG. 11 by using a single pump (SPV-2502 U) manufactured by Nippon Seimitu Co. so as to measure on the oxidized electrode in the samples containing various saccharides. A stainless steel block electrode was used as a counter electrode. 0.1 M phosphoric acid buffer solution (pH=6.8) was used as a mobile phase solution. Potential of 0.6 volt was applied to the glucose oxidase embodied electrode. The response curve was measured when the oxidized (anodically polarized ) glucose oxidase embodied electrode was used, and the resulting curve is shown in FIG. 14. Then, the response curve was measure by using the untreated glucose oxidase embodied electrode, and the resulting curve is shown in FIG. 15.

Ordinarily, the saccharides such as glucose, galactose and fructose can not be oxidized at the potential of 0.6 volt. However, in this experiment, glucose can be oxidized by the action of catalytic glucose oxidase because of the glucose oxidase embodied electrode, and the oxidizing current can be detected corresponding to the amount of the glucose therein. Then, the other saccharides can not be oxidized because it is not effected by the glucose oxidase, and the current change may not be caused even if the solution containing the other saccharides were put in. However, the not -treated (not-anodically polarized ) glucose oxidase embodied electrode caused the current based on the injection of galactose and fructose. Contrarily, there was not found the current change based on the injection of the other saccharide than glucose when the oxidation treated ( anodically treated) glucose oxidase embodied electrode was used.

### Industrial Utilization

Process of immobilizing of a biologically active substance, an element prepared thereby, and an analytical method using the said element are can be utilized for a variety of measurement and a measurement apparatus using a biologically active substance, and further, which element can function as a transducer or a reacting element, and are adapted for the measurement and the analytical apparatus with extremely small size, and extremely quick response and high sensitivity.

## Claims

1. A microbioelectrode comprising an electroconductive material, a porous conductive fine particle layer or porous conductive material layer made of platinum black of palladium black or fine particles of rhodium, iridium or gold and a biologically active substance selected from the group of enzyme, antibody, microorganism, organelle, antigen or hapten, adsorbed in said porous layer said microbioelectrode obtainable by the steps of:
a) electrochemically or electrolytically depositing the porous conductive fine particle layer or porous conductive material layer on the minute surface of the electroconductive material,
b) entrapping the biologically active substance, in said porous conductive fine particle layer or porous conductive material layer by immersing said layer into a solution containing said active substance.

2. The microbioelectrode according to claim 1, wherein step b) is followed by a washing step.

3. The microbioelectrode according to claim 1 or 2, wherein the electroconductive material is a wire having a diameter of 10 to 500 µm.

4. The microbioelectrode according to claim 3, wherein the wire is made of platinum.

5. The microbioelectrode according to claims 1 to 4, wherein further a polymeric film is formed on said porous conductive fine particle layer or porous conductive material layer.

6. The microbioelectrode according to claims 1 to 5, wherein said entrapped substance is further stabilized with a crosslinking agent.

7. The microbioelectrode according to claims 1 to 6, wherein said porous conductive fine particle layer or porous conductive material layer has been treated by anodic passivation.

8. A method of preparation of a microbioelectrode by the steps of:
a) electrochemically of electrolytically depositing on the surface of an electroconductive material a porous conductive material layer or porous conductive fine particle layer of platinum black, palladium black or fine particles of gold, rhodium or iridium and
b) immersing the resulting porous conductive material layer or porous conductive fine particle layer into a solution containing a biologically active substance selected from enzyme, antibody, microorganism, organelle, antigen or hapten, thereby adsorbing said active substance into said porous layer.

9. The method according to claim 8, wherein step b) is followed by washing.

10. The method according to claim 8 or 9, wherein the electroconductive material used in step a) is a wire having a diameter of 10 to 500 µm.

11. The method according to claim 10, wherein the wire is made of platinum.

12. The method according to claims 8 to 11, comprising further the step of forming a polymeric film on the surface of said layer.

13. The method in accordance with claims 8 to 12, comprising further the step of stabilizing the active substance incorporated into said layer by crosslinking.

14. The method according to claims 8 to 13, wherein said porous conductive fine particle layer or porous conductive material layer is treated by anodic passivation.

15. Use of the microbioelectrode according to claims 1 to 7 for an analytical method of determining a physiologically active substance, said method comprising:
1) measuring an electric signal generated when a pulsed potential is applied to said microbioelectrode as a functional electrode and
2) determining the concentration of the physiologically active substance as a target by said generated electric signal.

16. The use in accordance with claim 15, wherein a pulsed potential is applied to said microbioelectrode, and the current obtained as the transient response to the pulsed potential is measured for determining the concentration of the target substance.

## Patentansprüche

1. Mikrobiologische Elektrode mit einem elektrisch leitenden Material, einer porösen leitenden Feinpartikelschicht oder einer porösen leitenden Materialschicht aus Platinschwarz oder Palladiumschwarz oder feinen Partikeln aus Rhodium, Iridium oder Gold und einer biologisch aktiven Substanz, die aus der Gruppe der Enzyme, Antikörper, Mikroorganismen, Organelle, Antigenen oder Haptenen ausgewählt und in der porösen Schicht adsorbiert ist, erhältlich durch die folgenden Schritte:
a) Elektrochemisches oder elektrolytisches Abscheiden der porösen leitenden Feinpartikelschicht oder der porösen leitenden Materialschicht auf der feinen Oberfläche des elektrisch leitenden Materiales und
b) Einschließen der biologisch aktiven Substanz in der porösen leitenden Feinpartikelschicht oder der porösen leitenden Materialschicht durch Eintauchen der Schicht in eine die aktive Substanz enthaltende Lösung.

2. Mikrobiologische Elektrode nach Anspruch 1, bei der nach Schritt b) ein Waschschritt folgt.

3. Mikrobiologische Elektrode nach Anspruch 1 oder 2, bei der das elektrisch leitende Material ein Draht mit einem Durchmesser von 10 bis 500 µm ist.

4. Mikrobiologische Elektrode nach Anspruch 3, bei der der Draht aus Platin gefertigt ist.

5. Mikrobiologische Elektrode nach den Ansprüchen 1 bis 4, bei der des weiteren ein Polymerfilm aus der porösen leitenden Feinpartikelschicht oder der porösen leitenden Materialschicht ausgebildet ist.

6. Mikrobiologische Elektrode nach den Ansprüchen 1 bis 5, bei der die eingeschlossene Substanz des weiteren mit einem Vernetzungsmittel stabilisiert ist.

7. Mikrobiologische Elektrode nach den Ansprüchen 1 bis 6, bei der die poröse leitende Feinpartikelschicht oder die poröse leitende Materialschicht durch anodische Passivierung behandelt wurde.

8. Verfahren zur Herstellung einer mikrobiologischen Elektrode durch die folgenden Schritte:
a) Elektrochemisches oder elektrolytisches Abscheiden einer porösen leitenden Materialschicht oder einer porösen leitenden Feinpartikelschicht aus Platinschwarz, Palladiumschwarz oder feinen Partikeln aus Gold, Rhodium oder Iridium auf der Oberfläche eines elektrisch leitenden Materiales und
b) Eintauchen der entstandenen porösen leitenden Materialschicht oder porösen leitenden Feinpartikelschicht in eine Lösung, die eine biologisch aktive Substanz enthält, welche aus Enzymen, Antikörpern, Mikroorganismen, Organelle, Antigenen oder Haptenen ausgewählt ist und auf diese Weise Adsorbieren der aktiven Substanz in die poröse Schicht.

9. Verfahren nach Anspruch 8, bei dem nach Schritt b) ein Waschschritt folgt.

10. Verfahren nach Anspruch 8 oder 9, bei dem das in Schritt a) verwendete elektrisch leitende Material ein Draht mit einem Durchmesser von 10 bis 500 µm ist.

11. Verfahren nach Anspruch 10, bei dem der Draht aus Platin gefertigt ist.

12. Verfahren nach den Ansprüchen 8 bis 11, das des weiteren den Schritt der Ausbildung eines Polymerfilmes auf der Oberfläche der Schicht umfaßt.

13. Verfahren nach den Ansprüchen 8 bis 12, das des weiteren den Schritt der Stabilisierung der in die Schicht eingebauten aktiven Substanz durch Vernetzung umfaßt.

14. Verfahren nach den Ansprüche 8 bis 13, bei dem die poröse leitende Feinpartikelschicht oder die poröse leitende Materialschicht durch anodische Passivierung behandelt wird.

15. Verwendung der mikrobiologischen Elektrode nach den Ansprüchen 1 bis 7 für ein analytisches Verfahren zum Bestimmen einer physiologisch aktiven Substanz, wobei dieses Verfahren die folgenden Schritte umfaβt:
1) Messen eines elektrischen Signales, das erzeugt wird, wenn ein gepulstes Potential an die mikrobiologische Elektrode als Funktionselektrode angelegt wird, und
2) Bestimmen der Konzentration der physiologisch aktiven Substanz als Ziel durch das erzeugte elektrische Signal.

16. Verwendung nach Anspruch 15, bei der ein gepulstes Potential an die mikrobiologische Elektrode gelegt wird und der als Spitzenwert in bezug auf das gepulste Potential erhaltene Strom gemessen wird, um die Konzentration der Zielsubstanz zu bestimmen.

## Revendications

1. Une microbioélectrode, comprenant un matériau électro-conducteur, une couche poreuse composée de particules fines conductrices ou une couche poreuse en matériau conducteur, réalisée en noir de platine ou en noir de palladium ou en particules fines de rhodium, iridium ou or, et une substance biologiquement active choisie dans le groupe comprenant enzyme, anticorps, micro-organisme, organite, antigène ou haptène adsorbés dans ladite couche poreuse, ladite microbioélectrode étant susceptible d'être obtenue par les étapes consistant:
a) à déposer par voie électrochimique ou électrolytique la couche poreuse de particules fines conductrices ou la couche poreuse en matériau conducteur sur la surface de dimension réduite du matériau électro-conducteur;
b) à inclure la substance biologiquement active au sein de ladite couche poreuse en particules fines conductrices ou de ladite couche poreuse en matériau conducteur en immergeant ladite couche dans une solution qui contient ladite substance active.

2. La microbioélectrode selon la revendication 1, dans laquelle l'étape b) est suivie d'une étape de lavage.

3. La microbioélectrode selon la revendication 1 ou 2, dans laquelle le matériau électroconducteur est un fil présentant un diamètre de 10 à 500 micromètres.

4. La microbioélectrode selon la revendication 3, dans laquelle le fil est réalisé en platine.

5. La microbioélectrode selon les revendications 1 à 4, dans laquelle on forme, en outre, une couche polymère sur ladite couche poreuse en particules fines conductrices ou sur ladite couche poreuse en matériau conducteur.

6. La microbioélectrode selon les revendications 1 à 5, dans laquelle ladite substance incluse est en outre stabilisée par un agent de réticulation.

7. La microbioélectrode selon les revendications 1 à 6, dans laquelle ladite couche poreuse en particules fines conductrices ou ladite couche poreuse en matériau conducteur a subi un traitement de passivation anodique.

8. Un procédé de préparation d'une microbioélectrode comprenant les étapes consistant:
a) à déposer par voie électrochimique ou électrolytique sur la surface d'un matériau conducteur, une couche poreuse en matériau conducteur ou une couche poreuse en particules fines conductrices réalisée en noir de platine, en noir de palladium ou en particules fines d'or, de rhodium ou d'iridium; et
b) à immerger la couche poreuse résultante en matériau conducteur ou la couche poreuse en particules fines conductrices dans une solution qui contient une substance biologiquement active choisie dans le groupe comprenant enzyme, anticorps, micro-organisme, organite, antigène ou haptène, de manière à adsorber ladite substance active dans ladite couche poreuse.

9. Le procédé selon la revendication 8, dans lequel l'étape (b) est suivie d'une étape de lavage.

10. Le procédé selon la revendication 8 ou 9, dans lequel le matériau électro-conducteur utilisé à l'étape (a) est un fil présentant un diamètre de 10 à 500 micromètres.

11. Le procédé selon la revendication 10, dans lequel le fil est réalisé en platine.

12. Le procédé selon les revendications 8 à 11, comprenant en outre l'étape consistant à former un film polymére sur la surface de ladite couche.

13. Le procédé selon les revendications 8 à 12, comprenant en outre l'étape consistant à stabiliser la substance active incorporée dans ladite couche par réticulation.

14. Le procédé selon les revendications 8 à 13, dans lequel ladite couche poreuse en particules fines conductrices ou ladite couche poreuse en matériau conducteur est traitée par passivation anodique.

15. L'utilisation de la microbioélectrode selon les revendications 1 à 7, pour un procédé analytique de détermination d'une substance physiologiquement active, ledit procédé consistant:
1) à mesurer un signal électrique émis lorsqu'un potentiel pulsé est appliqué à ladite microbioélectrode en tant qu'électrode fonctionnelle et
2) à déterminer la concentration de la substance physiologiquement active en tant que consigne, au moyen dudit signal émis électriquement.

16. L'utilisation selon la revendication 15, dans laquelle on applique un potentiel pulsé à ladite microbioélectrode et on mesure le courant obtenu comme réponse transitoire au potentiel pulsé, pour déterminer la concentration de la substance de consigne.
